# EUROPEAN PATENT APPLICATION

(11) **EP 4 692 802 A1**
(43) Date of publication of application: **11.02.2026**
(21) Application number: 24780766.2
(22) Date of filing: 29.03.2024
(51) Int. Cl.: G01N 33/68, C12N 15/25, C12N 15/28, C12Q 1/686, C12Q 1/6851, C12Q 1/6869

(54) **BIOMARKER FOR PREDICTING PROGNOSIS IN SEPTIC PATIENT AND USE THEREOF**

(30) Priority: 31.03.2023 JP 2023058005
(71) Applicant: The University of Osaka, Osaka 565-0871 (JP); CHUGAI SEIYAKU KABUSHIKI KAISHA, Tokyo 115-8543 (JP)
(72) Inventor: MATSUMOTO, Hisatake, Suita-shi, Osaka 565-0871 (JP); TERAO, Kimio, Tokyo 103-8324 (JP); MATSUNAGA, Kiyoshi, Tokyo 103-8324 (JP); NAKAMURA, Mikiko, Tokyo 103-8324 (JP); SHIMADA, Sumire, Tokyo 103-8324 (JP)
(74) Representative: Vossius & Partner Patentanwälte Rechtsanwälte mbB
(86) International application number: PCT/JP2024/012956
(87) International publication number: WO 2024/204671

(57) **Abstract**

The present invention provides a biomarker for predicting a prognosis of a septic patient, the biomarker comprising IL-1β and TNF-α, wherein a blood concentration ratio of IL-1β to TNF-α is an indicator for predicting a prognosis of the septic patient.

## Description

### TECHNICAL FIELD

The present invention relates to a method for predicting a prognosis of a septic patient, a method for predicting a therapeutic effect of an IL-6 inhibitor in a septic patient, and a method for selecting a septic patient who is expected to obtain a therapeutic effect of an IL-6 inhibitor, and biomarkers for them, and the like.

### BACKGROUND ART

Sepsis is a condition in which the biological response associated with an infection becomes unregulated in vivo and can cause life-threatening organ damages. Septic shock is a category included in sepsis and defined as a condition in which acute circulatory failure causes severe cellular disorders and metabolic abnormalities and the risk of death is increased compared to sepsis without shock (Non Patent Literatures 1 and 2). Septic shock is caused by cytokine storm that is an excessive immune response.

Since an increase in plasma IL-6 concentration in septic patients was reported in 1989, multiple reports have been made about the therapeutic effect of IL-6 inhibition on sepsis. Patent Literature 1 discloses that administration of monoclonal antibodies to IL-6 may prevent septic shock. Patent Literature 2 discloses that anti-IL-6 receptor antibodies inhibit proteolytic degradation that occurs during sepsis. Patent Literature 3 lists sepsis as an IL-6-related disease that can be treated with an IL-6 inhibitor such as an anti-IL-6 receptor antibody. Non Patent Literature 3 suggests the effect of tocilizumab, a humanized anti-IL-6 receptor monoclonal antibody, on the control of cytokine storm during sepsis, and discloses that tocilizumab is promising as a sepsis therapeutic agent. Non Patent Literature 4 discloses the role of IL-6 inhibitors in the treatment of cytokine storm (also known as cytokine release syndrome) associated with COVID-19.

Biomarkers may be useful for diagnosing a patient with sepsis and for identifying a patient for whom certain treatments may be effective. Various biomarkers for sepsis have been reported so far (Non Patent Literature 5, Tables 1 to 3, and the like). For example, biomarkers of cytokines/chemokines, such as IL-1β, IL-2, IL-4, IL-6, IL-8, MIP-1, MIP-2, MCP-1 and MCP-2, biomarkers of cell markers such as CD10, CD14 and CD18, and biomarkers of receptors, such as CCR3, C5L2, FLT-1, GP130, Toll-like receptor and TNF receptor, are known. Patent Literature 4 discloses a method for detecting early sepsis, comprising the step of monitoring expression of markers including neutrophil-associated CD11b, neutrophil-associated CD64, and monocyte-associated HLA-DR. Patent Literature 5 discloses a biomarker for identifying cytokine release syndrome and sepsis (e.g., Example 4). Patent Literature 6 discloses a marker for differential diagnosis and a method of use thereof, and describes that the concentration of thrombus precursor protein is elevated in sepsis, that elevated serum concentration of MCP-1 is associated with sepsis, that plasma procalcitonin is identified as a marker of sepsis and its severity, and that overexpression of IL-10 is identified as a marker in sepsis.

It has been proposed that the production of inflammatory cytokines such as TNFα and IL-1β in infectious diseases involves two production mechanisms of the signalosome pathway and the inflammasome pathway (Non Patent Literature 6). Signalosome is a protein complex that transmits signals from receptors that recognize microbial-specific molecular patterns into cells and activates NFκB to increase the production of inflammatory cytokines. Inflammasome is a protein complex that is formed in response to cellular disorders and changes the inactive form procaspase-1 to the active form caspase-1. Caspase-1 cleaves pro-IL-1β intracellularly to produce IL-1β, and IL-1β is secreted extracellularly to function as an inflammatory cytokine. Although TNFα and IL-1β production is increased in both pathways, the inflammasome pathway is thought to increase IL-1β more significantly than the signalosome pathway.

### CITATION LIST

### PATENT LITERATURE

PTL 1: International Publication No. WO1991/007986
PTL 2: Japanese Patent No. 4540132
PTL 3: Japanese Patent No. 6130983
PTL 4: Japanese Patent No. 4520157
PTL 5: International Publication No. WO2017/040930
PTL 6: International Publication No. WO2004/059293

### NON PATENT LITERATURE

NPL 1: The Japanese Clinical Practice Guidelines for Management of Sepsis and Septic Shock 2020 (J-SSCG 2020); The Journal of the Japanese Society of Intensive Care Medicine Vol. 28 Supplement, February 25, 2021
NPL 2: JAMA 2016; 315:801-10
NPL 3: Pharmazie. 2016 Nov 2; 71(11):636-639
NPL 4: Int J Med Sci. 2021; 18(6) 1356-1362
NPL 5: Critical Care 2010; 14:R15
NPL 6: The journal of the Japanese Society for Clinical Microbiology, Vol.26, No.3, p. 15-28 2016

### SUMMARY OF INVENTION

### TECHNICAL PROBLEM

Various markers for sepsis have been reported so far, but no markers have been known to predict the prognosis of septic patients. If markers for predicting prognosis are established, it will be possible to provide appropriate treatments to septic patients at an early stage.

Accordingly, it is an object of the present invention to provide a biomarker for predicting the prognosis of septic patients, a method for selecting septic patients using the biomarker, and the like.

### SOLUTION TO PROBLEM

The present inventors have intensively studied, and as a result, have found that the blood concentration ratio of IL-1 β to TNF-α in septic patients is useful as a marker for predicting the prognosis of sepsis. Although TNFα and IL-1β have been known as inflammatory cytokines, it has not been known that their blood concentration ratio could be a marker for predicting the prognosis of septic patients. The present inventors have further studied and completed the present invention.

That is, the present invention provides the following invention.
[A1] A biomarker for predicting a prognosis of a septic patient, the biomarker comprising IL-1β and TNF-α, wherein
   a blood concentration ratio of IL-1β to TNF-α is an indicator for predicting a prognosis of the septic patient.
[A2] The biomarker according to A1, wherein the blood concentration ratio of 1 or more is an indicator of poor prognosis of the septic patient.
[A3] The biomarker according to A1 or A2, wherein the blood concentration ratio is a blood concentration ratio of IL-1β to TNF-α within 2 days, preferably within 1 day, from hospitalization, from an onset of sepsis, or from being diagnosed with sepsis.
[A4] The biomarker according to any of A1 to A3, wherein blood concentrations of IL-1β and TNF-α are concentrations of IL-1β and TNF-α in serum, plasma, or whole blood obtained from the septic patient.
[A5] The biomarker according to any of A1 to A4, wherein the blood concentrations of IL-1β and TNF-α are measured by a Proximity Extension Assay (PEA) method.
[A6] The biomarker according to A5, wherein a measured value by the PEA method is an expression value of a protein obtained using a next-generation sequencer or quantitative real-time PCR.
[A7] The biomarker according to any of A1 to A6, wherein the septic patient is a human.
[A8] The biomarker according to any of A1 to A7, wherein the septic patient has not received a treatment with an IL-6 inhibitor.
[A9] The biomarker according to any of A1 to A8, wherein the prognosis is a life prognosis.
[B1] A method for predicting a prognosis of a septic patient, the method comprising
   a step of measuring concentrations of IL-1β and TNF-α in a blood sample obtained from a septic patient, wherein
   a blood concentration ratio of IL-1β to TNF-α is an indicator for predicting a prognosis of the septic patient.
[B2] The method according to B1, wherein the blood concentration ratio of 1 or more is an indicator of poor prognosis of the septic patient.
[B3] The method according to B1 or B2, wherein the blood concentration ratio is a blood concentration ratio of IL-1β to TNF-α within 2 days, preferably within 1 day, from hospitalization, from an onset of sepsis, or from being diagnosed with sepsis.
[B4] The method according to any of B1 to B3, wherein the blood sample is serum, plasma, or whole blood.
[B5] The method according to any of B1 to B4, wherein the blood concentrations of IL-1β and TNF-α are measured by a Proximity Extension Assay (PEA) method.
[B6] The method according to B5, wherein a measured value by the PEA method is an expression value of a protein obtained using a next-generation sequencer or quantitative real-time PCR.
[B7] The method according to any of B1 to B6, wherein the septic patient is a human.
[B8] The method according to any of B1 to B7, wherein the septic patient has not received a treatment with an IL-6 inhibitor.
[B9] The method according to any of B1 to B8, wherein the prognosis is a life prognosis.
[C1] A biomarker for predicting a therapeutic effect of an IL-6 inhibitor in a septic patient, the biomarker comprising IL-1β and TNF-α, wherein
   a blood concentration ratio of IL-1β to TNF-α is an indicator for predicting a therapeutic effect of an IL-6 inhibitor in the septic patient.
[C2] The biomarker according to C1, wherein the blood concentration ratio of 1 or more is an indicator that the septic patient is expected to obtain a therapeutic effect of an IL-6 inhibitor.
[C3] The biomarker according to C1 or C2, wherein the blood concentration ratio is a blood concentration ratio of IL-1β to TNF-α within 2 days, preferably within 1 day, from hospitalization, from an onset of sepsis, or from being diagnosed with sepsis.
[C4] The biomarker according to any of C1 to C3, wherein blood concentrations of IL-1β and TNF-α are concentrations of IL-1β and TNF-α in serum, plasma, or whole blood obtained from the septic patient.
[C5] The biomarker according to any of C1 to C4, wherein the blood concentrations of IL-1β and TNF-α are measured by a Proximity Extension Assay (PEA) method.
[C6] The biomarker according to C5, wherein a measured value by the PEA method is an expression value of a protein obtained using a next-generation sequencer or quantitative real-time PCR.
[C7] The biomarker according to any of C1 to C6, wherein the septic patient is a human.
[C8] The biomarker according to any of C1 to C7, wherein the septic patient has not received a treatment with an IL-6 inhibitor.
[C9] The biomarker according to any of C1 to C8, wherein the IL-6 inhibitor is an IL-6 receptor inhibitor.
[D1] A method for predicting a therapeutic effect of an IL-6 inhibitor in a septic patient, the method comprising
   a step of measuring concentrations of IL-1β and TNF-α in a blood sample obtained from a septic patient, wherein
   a blood concentration ratio of IL-1β to TNF-α is an indicator for predicting a therapeutic effect of an IL-6 inhibitor in the septic patient.
[D2] The method according to D1, wherein the blood concentration ratio of 1 or more is an indicator that the septic patient is expected to obtain a therapeutic effect of an IL-6 inhibitor.
[D3] The method according to D1 or D2, wherein the blood concentration ratio is a blood concentration ratio of IL-1β to TNF-α within 2 days, preferably within 1 day, from hospitalization, from an onset of sepsis, or from being diagnosed with sepsis.
[D4] The method according to any of D1 to D3, wherein the blood sample is serum, plasma, or whole blood.
[D5] The method according to any of D1 to D4, wherein the blood concentrations of IL-1β and TNF-α are measured by a Proximity Extension Assay (PEA) method.
[D6] The method according to D5, wherein a measured value by the PEA method is an expression value of a protein obtained using a next-generation sequencer or quantitative real-time PCR.
[D7] The method according to any of D1 to D6, wherein the septic patient is a human.
[D8] The method according to any of D1 to D7, wherein the septic patient has not received a treatment with an IL-6 inhibitor.
[D9] The method according to any of D1 to D8, wherein the IL-6 inhibitor is an IL-6 receptor inhibitor.
[E1] A biomarker for selecting a septic patient who is expected to obtain a therapeutic effect of an IL-6 inhibitor, the biomarker comprising IL-1β and TNF-α, wherein
   a blood concentration ratio of IL-1β to TNF-α is an indicator that the septic patient is expected to obtain a therapeutic effect of an IL-6 inhibitor.
[E2] The biomarker according to E1, wherein the blood concentration ratio of 1 or more is an indicator that the septic patient is expected to obtain a therapeutic effect of an IL-6 inhibitor.
[E3] The biomarker according to E1 or E2, wherein the blood concentration ratio is a blood concentration ratio of IL-1β to TNF-α within 2 days, preferably within 1 day, from hospitalization, from an onset of sepsis, or from being diagnosed with sepsis.
[E4] The biomarker according to any of E1 to E3, wherein blood concentrations of IL-1β and TNF-α are concentrations of IL-1β and TNF-α in serum, plasma, or whole blood obtained from the septic patient.
[E5] The biomarker according to any of E1 to E4, wherein the blood concentrations of IL-1β and TNF-α are measured by a Proximity Extension Assay (PEA) method.
[E6] The biomarker according to E5, wherein a measured value by the PEA method is an expression value of a protein obtained using a next-generation sequencer or quantitative real-time PCR.
[E7] The biomarker according to any of E1 to E6, wherein the septic patient is a human.
[E8] The biomarker according to any of E1 to E7, wherein the septic patient has not received a treatment with an IL-6 inhibitor.
[E9] The biomarker according to any of E1 to E8, wherein the IL-6 inhibitor is an IL-6 receptor inhibitor.
[F1] A method for selecting a septic patient who is expected to obtain a therapeutic effect of an IL-6 inhibitor, the method comprising
   a step of measuring concentrations of IL-1β and TNF-α in a blood sample obtained from a septic patient, wherein
   a blood concentration ratio of IL-1β to TNF-α is an indicator for predicting that the septic patient is expected to obtain a therapeutic effect of an IL-6 inhibitor.
[F2] The method according to F1, wherein the blood concentration ratio of 1 or more is an indicator that the septic patient is expected to obtain a therapeutic effect of an IL-6 inhibitor.
[F3] The method according to F1 or F2, wherein the blood concentration ratio is a blood concentration ratio of IL-1β to TNF-α within 2 days, preferably within 1 day, from hospitalization, from an onset of sepsis, or from being diagnosed with sepsis.
[F4] The method according to any of F1 to F3, wherein the blood sample is serum, plasma, or whole blood.
[F5] The method according to any of F1 to F4, wherein the blood concentrations of IL-1β and TNF-α are measured by a Proximity Extension Assay (PEA) method.
[F6] The method according to F5, wherein a measured value by the PEA method is an expression value of a protein obtained using a next-generation sequencer or quantitative real-time PCR.
[F7] The method according to any of F1 to F6, wherein the septic patient is a human.
[F8] The method according to any of F1 to F7, wherein the septic patient has not received a treatment with an IL-6 inhibitor.
[F9] The method according to any of F1 to F8, wherein the IL-6 inhibitor is an IL-6 receptor inhibitor.
[F10] The method according to any of F1 to F9, further comprising a step of calculating a blood concentration ratio of IL-1β to TNF-α from a measurement result obtained in the step of measuring concentrations of IL-1β and TNF-α in a blood sample obtained from a septic patient, and selecting a septic patient who is expected to obtain a therapeutic effect of an IL-6 inhibitor based on the blood concentration ratio.
[G1] A therapeutic agent for sepsis, the therapeutic agent comprising
   an IL-6 inhibitor as an active ingredient, wherein
   the therapeutic agent is used to be administered to a septic patient selected based on a blood concentration ratio of IL-1β to TNF-α, who is expected to obtain a therapeutic effect of an IL-6 inhibitor.
[G2] The therapeutic agent according to G1, wherein the blood concentration ratio of 1 or more is an indicator that the septic patient is expected to obtain a therapeutic effect of an IL-6 inhibitor.
[G3] The therapeutic agent according to G1 or G2, wherein the blood concentration ratio is a blood concentration ratio of IL-1β to TNF-α within 2 days, preferably within 1 day, from hospitalization, from an onset of sepsis, or from being diagnosed with sepsis.
[G4] The therapeutic agent according to any of G1 to G3, wherein blood concentrations of IL-1β and TNF-α are concentrations of IL-1β and TNF-α in serum, plasma, or whole blood obtained from the septic patient.
[G5] The therapeutic agent according to any of G1 to G4, wherein the blood concentrations of IL-1β and TNF-α are measured by a Proximity Extension Assay (PEA) method.
[G6] The therapeutic agent according to G5, wherein a measured value by the PEA method is an expression value of a protein obtained using a next-generation sequencer or quantitative real-time PCR.
[G7] The therapeutic agent according to any of G1 to G6, wherein the septic patient is a human.
[G8] The therapeutic agent according to any of G1 to G7, wherein the septic patient has not received a treatment with an IL-6 inhibitor.
[G9] The therapeutic agent according to any of G1 to G8, wherein the IL-6 inhibitor is an IL-6 receptor inhibitor.
[H1] A method for treating sepsis, the method comprising
   administering an IL-6 inhibitor to a septic patient, wherein
   the septic patient is a septic patient selected based on a blood concentration ratio of IL-1β to TNF-α, who is expected to obtain a therapeutic effect of an IL-6 inhibitor.
[H2] The method according to H1, wherein the blood concentration ratio of 1 or more is an indicator that the septic patient is expected to obtain a therapeutic effect of an IL-6 inhibitor.
[H3] The method according to H1 or H2, wherein the blood concentration ratio is a blood concentration ratio of IL-1β to TNF-α within 2 days, preferably within 1 day, from hospitalization, from an onset of sepsis, or from being diagnosed with sepsis.
[H4] The method according to any of H1 to H3, wherein the blood sample is serum, plasma, or whole blood.
[H5] The method according to any of H1 to H4, wherein blood concentrations of IL-1β and TNF-α are measured by a Proximity Extension Assay (PEA) method.
[H6] The method according to H5, wherein a measured value by the PEA method is an expression value of a protein obtained using a next-generation sequencer or quantitative real-time PCR.
[H7] The method according to any of H1 to H6, wherein the septic patient is a human.
[H8] The method according to any of H1 to H7, wherein the septic patient has not received a treatment with an IL-6 inhibitor.
[H9] The method according to any of H1 to H8, wherein the IL-6 inhibitor is an IL-6 receptor inhibitor.
[H10] The method according to any of H1 to H9, further comprising a step of measuring concentrations of IL-1β and TNF-α in a blood sample obtained from the septic patient.
[H11] The method according to H10, further comprising a step of calculating a blood concentration ratio of IL-1β to TNF-α from a measurement result obtained in the step of measuring concentrations of IL-1β and TNF-α in a blood sample obtained from the septic patient, and selecting a septic patient who is expected to obtain a therapeutic effect of an IL-6 inhibitor based on the blood concentration ratio.
[I1] A suppressor of cytokine storm associated with sepsis, the suppressor comprising
   an IL-6 inhibitor as an active ingredient, wherein
   the suppressor is used to be administered to a septic patient selected based on a blood concentration ratio of IL-1β to TNF-α, who is expected to obtain a therapeutic effect of an IL-6 inhibitor.
[I2] The suppressor according to I1, wherein the blood concentration ratio of 1 or more is an indicator that the septic patient is expected to obtain a therapeutic effect of an IL-6 inhibitor.
[I3] The suppressor according to I1 or I2, wherein the blood concentration ratio is a blood concentration ratio of IL-1β to TNF-α within 2 days, preferably within 1 day, from hospitalization, from an onset of sepsis, or from being diagnosed with sepsis.
[I4] The suppressor according to any of I1 to I3, wherein blood concentrations of IL-1β and TNF-α are concentrations of IL-1β and TNF-α in serum, plasma, or whole blood obtained from the septic patient.
[I5] The suppressor according to any of I1 to I4, wherein the blood concentrations of IL-1β and TNF-α are measured by a Proximity Extension Assay (PEA) method.
[I6] The suppressor according to I5, wherein a measured value by the PEA method is an expression value of a protein obtained using a next-generation sequencer or quantitative real-time PCR.
[I7] The suppressor according to any of I1 to I6, wherein the septic patient is a human.
[I8] The suppressor according to any of I1 to I7, wherein the septic patient has not received a treatment with an IL-6 inhibitor.
[I9] The suppressor according to any of I1 to I8, wherein the IL-6 inhibitor is an IL-6 receptor inhibitor.
[J1] A method for suppressing cytokine storm associated with sepsis, the method comprising
   administering an IL-6 inhibitor to a septic patient, wherein
   the septic patient is a septic patient selected based on a blood concentration ratio of IL-1β to TNF-α, who is expected to obtain a therapeutic effect of an IL-6 inhibitor.
[J2] The method according to J1, wherein the blood concentration ratio of 1 or more is an indicator that the septic patient is expected to obtain a therapeutic effect of an IL-6 inhibitor.
[J3] The method according to J1 or J2, wherein the blood concentration ratio is a blood concentration ratio of IL-1β to TNF-α within 2 days, preferably within 1 day, from hospitalization, from an onset of sepsis, or from being diagnosed with sepsis.
[J4] The method according to any of J1 to J3, wherein a blood sample is serum, plasma, or whole blood.
[J5] The method according to any of J1 to J4, wherein blood concentrations of IL-1β and TNF-α are measured by a Proximity Extension Assay (PEA) method.
[J6] The method according to J5, wherein a measured value by the PEA method is an expression value of a protein obtained using a next-generation sequencer or quantitative real-time PCR.
[J7] The method according to any of J1 to J6, wherein the septic patient is a human.
[J8] The method according to any of J1 to J7, wherein the septic patient has not received a treatment with an IL-6 inhibitor.
[J9] The method according to any of J1 to J8, wherein the IL-6 inhibitor is an IL-6 receptor inhibitor.
[J10] The method according to any of J1 to J9, further comprising a step of measuring concentrations of IL-1 β and TNF-α in a blood sample obtained from the septic patient.
[J11] The method according to J10, further comprising a step of calculating a blood concentration ratio of IL-1β to TNF-α from a measurement result obtained in the step of measuring concentrations of IL-1β and TNF-α in a blood sample obtained from the septic patient, and selecting a septic patient who is expected to obtain a therapeutic effect of an IL-6 inhibitor based on the blood concentration ratio.
[K1] A kit for predicting a prognosis (preferably a life prognosis) of a septic patient, for predicting a therapeutic effect of an IL-6 inhibitor in a septic patient, or for selecting a septic patient who is expected to obtain a therapeutic effect of an IL-6 inhibitor, the kit comprising a reagent for measuring blood concentrations of IL-1β and TNF-α and calculating a blood concentration ratio of IL-1β to TNF-α.
[K2] The kit according to K1, further comprising an instruction describing a method for predicting a prognosis of a septic patient, a method for predicting a therapeutic effect of an IL-6 inhibitor in a septic patient, or a method for selecting a septic patient who is expected to obtain a therapeutic effect of an IL-6 inhibitor, from a blood concentration ratio of IL-1β to TNF-α calculated from blood concentrations of IL-1β and TNF-α.
[K3] The kit according to K2, wherein the instruction states that when the blood concentration ratio of IL-1β to TNF-α in a sample obtained from the septic patient is higher than a preset predetermined value, it is determined that a prognosis of the septic patient is poor or the septic patient is expected to obtain a therapeutic effect of an IL-6 inhibitor.
[K4] The kit according to any of K1 to K3, wherein the blood concentration ratio of 1 or more is an indicator that a prognosis of the septic patient is poor or the septic patient is expected to obtain a therapeutic effect of an IL-6 inhibitor.
[K5] The kit according to any of K1 to K4, wherein the blood concentration ratio is a blood concentration ratio of IL-1β to TNF-α within 2 days, preferably within 1 day, from hospitalization, from an onset of sepsis, or from being diagnosed with sepsis.
[K6] The kit according to any of K1 to K5, wherein the blood concentrations of IL-1β and TNF-α are concentrations of IL-1β and TNF-α in serum, plasma, or whole blood obtained from the septic patient.
[K7] The kit according to any of K1 to K6, wherein the blood concentrations of IL-1β and TNF-α are measured by a Proximity Extension Assay (PEA) method.
[K8] The kit according to K7, wherein a measured value by the PEA method is an expression value of a protein obtained using a next-generation sequencer or quantitative real-time PCR.
[K9] The kit according to any of K1 to K8, wherein the septic patient is a human.
[K10] The kit according to any of K1 to K9, wherein the septic patient has not received a treatment with an IL-6 inhibitor.

### ADVANTAGEOUS EFFECTS OF INVENTION

According to the present invention, it is possible to identify in a septic patient at an early stage a patient who is predicted to have a poor prognosis or who is expected to obtain a therapeutic effect of an IL-6 inhibitor, and to provide an appropriate treatment to the septic patient.

### BRIEF DESCRIPTION OF DRAWINGS

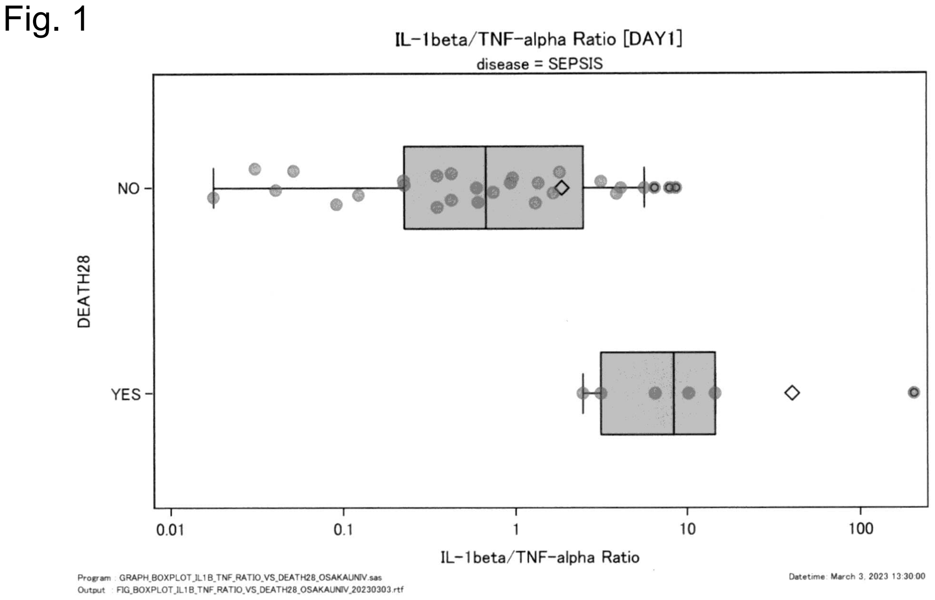

Fig. 1 is a diagram showing the distribution of the blood concentration ratio of IL-1 β to TNF-α (IL-1β/TNF-α) on day 1 of hospitalization in septic patients. Y and N show patients who died and survived within 28 days after hospitalization, respectively (Y: n = 6; N: n = 28).

### DESCRIPTION OF EMBODIMENTS

### 1. Definitions

The "sepsis" as used herein refers to "a condition in which severe organ damage is caused by an infection" as defined in the Japanese Clinical Practice Guidelines for Management of Sepsis and Septic Shock 2020 (J-SSCG 2020) (The Journal of the Japanese Society of Intensive Care Medicine Vol. 28 Supplement, February 25, 2021). The "septic shock" as used herein refers to "a condition in which acute circulatory failure causes severe cellular disorders and metabolic abnormalities and the risk of death is increased compared to sepsis without shock" as defined in J-SSCG 2020. These definitions described in J-SSCG 2020 are based on "The Third International Consensus Definitions for Sepsis and Septic Shock (Sepsis-3)" (JAMA 2016; 315:801-10) published in February 2016.

The "IL-1β" is a representative inflammatory cytokine along with TNFα and IL-6. IL-1β is encoded by the IL1B gene in humans and by the Il1b gene in mice. It is known that IL-1β is mainly produced by monocytes, macrophages, and dendritic cells, and is also secreted in small amounts from B lymphocytes and NK cells. IL-1β is produced by cleavage of intracellular pro-IL-1β by caspase-1 activated by a protein complex called inflammasome, and secreted extracellularly. IL-1β is thought to function as an inflammatory cytokine.

The "TNF-α", also called a tumor necrosis factor, is an inflammatory cytokine. TNF-α is mainly produced by macrophages during inflammation caused by infection or the like, and is involved in infection defense. However, excess production of TNF-α in sepsis can cause septic shock.

The "IL-6 inhibitor" as used herein is a substance that blocks signaling by IL-6 and inhibits the biological activity of IL-6. Specific examples of the IL-6 inhibitor can include a substance that binds to IL-6, a substance that binds to IL-6 receptors, and a substance that binds to gp130. Other examples of the IL-6 inhibitor can include a substance that inhibits STAT3 phosphorylation, which is important as intracellular signal by IL-6, such as AG490. The IL-6 inhibitor includes, but are not particularly limited to, an anti-IL-6 antibody (including a chimeric anti-IL-6 antibody, a humanized anti-IL-6 antibody, a human anti-IL-6 antibody), an anti-IL-6 receptor antibody (including a chimeric anti-IL-6R antibody, a humanized anti-IL-6R antibody, a human anti-IL-6R antibody), an anti-gp130 antibody, a modified IL-6, a soluble modified IL-6 receptor, an IL-6 partial peptide, an IL-6 receptor partial peptide, and a low molecular compound exhibiting similar activity to these. Examples of the anti-IL-6 antibody include Siltuximab, Olokizumab (CDP6038), Elsilimomab, Sirukumab (CNTO136), Clazakizumab (ALD518, BMS-945429), Gerilimzumab (ARGX 109), and FM101. Examples of the anti-IL-6R antibody include Tocilizumab, Satralizumab, and Sarilumab. Examples of the low molecular compound include CPSI-2364. Preferred embodiments of the IL-6 inhibitor can include an IL-6 receptor inhibitor, particularly an anti-IL-6 receptor antibody.

The "IL-6 receptor inhibitor" as used herein is a substance that blocks signaling via an IL-6 receptor and inhibits the biological activity of an IL-6 receptor. The IL-6 receptor inhibitor may be a substance that binds to an IL-6 receptor and directly inhibits the biological activity of the IL-6 receptor, or may be a substance that binds to other substances such as gp130 and indirectly inhibits the biological activity of an IL-6 receptor, but is preferably a substance that binds to the IL-6 receptor and has an activity that inhibits the binding of IL-6 to an IL-6 receptor.

Examples of the IL-6 receptor inhibitor used in the present invention include, but are not particularly limited to, an anti-IL-6 receptor antibody, a soluble modified IL-6 receptor, a partial peptide of IL-6 receptor, and a low molecular substance exhibiting similar activity to these. Preferred examples of the IL-6 receptor inhibitor used in the present invention can include an antibody that recognizes an IL-6 receptor.

The origin of the anti-IL-6 receptor antibodies used in the present invention is not particularly limited, but antibodies derived from mammals are preferred.

The anti-IL-6 receptor antibodies used in the present invention can be obtained as polyclonal or monoclonal antibodies using known means. As the anti-IL-6 receptor antibodies used in the present invention, monoclonal antibodies derived from mammals are particularly preferred. The monoclonal antibodies derived from mammals include those produced in hybridomas and those produced in hosts transformed with an expression vector containing an antibody gene by genetic engineering techniques. The antibodies inhibit the binding of IL-6 to an IL-6 receptor by binding to the IL-6 receptor, and blocks the transmission of IL-6 biological activity into cells.

Examples of such antibodies include MR16-1 antibodies (Tamura, T. et al. Proc. Natl. Acad. Sci. USA (1993) 90, 11924-11928); PM-1 antibody (Hirata, Y. et al., J. Immunol. (1989) 143, 2900-2906), AUK12-20 antibody, AUK64-7 antibody or AUK146-15 antibody (International Publication No. WO 92-19759), tocilizumab, and satralizumab. Among these, preferred monoclonal antibodies to human IL-6 receptors include, but are not limited to, a PM-1 antibody, tocilizumab, and satralizumab.

The anti-IL-6 receptor antibodies can be manufactured using known techniques as disclosed in WO 2016/195088 A1 and the like.

The antibodies used in the present invention may be a fragment or a modified body of an antibody as long as it can be suitably used in the present invention. Examples of the fragment of an antibody include Fab, F(ab')₂, Fv, or a single chain Fv (scFv) linking Fv of the H chain and Fv of the L chain with an appropriate linker. These can be manufactured using known techniques as disclosed in WO 2016/195088 A1 and the like.

### 2. Biomarker for predicting prognosis of septic patient and use thereof

In one aspect, the present invention relates to a biomarker for predicting a prognosis of a septic patient. The biomarker according to this aspect (hereinafter also referred to as the biomarker I of the present invention) comprises IL-1β and TNF-α, wherein a blood concentration ratio of IL-1β to TNF-α is an indicator for predicting a prognosis of the septic patient.

As used herein, the "prognosis" of a septic patient refers to the course or prospect predicted after the onset of sepsis. Poor prognosis or unfavorable prognosis means that a septic patient is more likely to die at the time of predicting the prognosis. Good prognosis or favorable prognosis means that a septic patient is predicted to be less likely to die at the time of predicting the prognosis.

In one embodiment, the "prognosis" may be the likelihood of death after a certain period from the time of onset or diagnosis of sepsis, or hospitalization. In this case, the "prognosis" is also called life prognosis. In certain embodiments, the "prognosis" may be death within 28 days from the time of hospitalization. Day-28 death (Death28) is commonly used as a clinical outcome for sepsis (The Japanese Clinical Practice Guidelines for Management of Sepsis and Septic Shock 2020 (J-SSCG 2020); The Journal of the Japanese Society of Intensive Care Medicine Vol. 28 Supplement, February 25, 2021).

The blood concentration ratio of IL-1β to TNF-α can be calculated from the values obtained by measuring the concentrations of IL-1β and TNF-α in a blood sample obtained from the septic patient. The blood sample may be serum, plasma, or whole blood, and preferably serum. The methods for obtaining a blood sample from human are well known to those skilled in the art. It should be noted that a blood sample processed in some way may also be included in the blood sample herein.

The timing of taking the blood sample from a patient is not particularly limited, but it is preferable that the elapsed time from the time of onset or diagnosis of sepsis, or hospitalization is short. In one embodiment, the blood sample may be taken within 2 days, preferably within 1 day, from hospitalization, within 2 days, preferably within 1 day, from onset of sepsis, or within 2 days, preferably within 1 day, from diagnosis of sepsis (including diagnosis of possible sepsis). In a preferred embodiment, a blood sample may be taken within 2 days, preferably within 1 day, from hospitalization.

The measurement of the blood concentrations of IL-1β and TNF-α can be performed by techniques known to those skilled in the art as a method of measuring concentrations of cytokines. Examples of such methods include enzyme immunoassays (ELISA, EIA), fluorescence immunoassays (FIA), radioimmunoassays (RIA), luminescence immunoassays (LIA), electrochemiluminescence (ECL) methods, western blotting methods, surface plasmon resonance methods, methods using antibody arrays, immunohistostaining methods, fluorescence activated cell sorting (FACS) methods, immunochromatography methods, immunoprecipitation methods, immunonephelometry methods, latex agglutination methods, and Proximity Extension Assay (PEA) methods (Olink, White Paper "PEA - a high-multiplex immunoassay technology with qPCR or NGS readout" (https://olink.com/resources-support/document-download-center/#whitepapers) and E. Assarsson et al., Homogenous 96-plex PEA immunoassay exhibiting high sensitivity, specificity, and excellent scalability. PLOS ONE 9, e95192 (2014)). In preferred embodiments, the blood concentrations of IL-1β and TNF-α may be measured by a PEA method. Measured values by the PEA method can be obtained as protein expression values (e.g., Normalized Protein eXpression (NPX)) using a next-generation sequencer such as NovaSeq (Illumina, Inc.) or quantitative real-time PCR (qPCR), and analytical software such as NPX Explore software (Olink proteomics).

In sepsis, there are patients who die from a state of septic shock or the like, and patients who recover and survive without such a state. In the past, there has been no way to identify these patients at an early stage. The present inventors have found for the first time that there is a difference in the blood concentration ratio of IL-1β to TNF-α between the deceased patient group and the surviving patient group. Thus, the blood concentration ratio of IL-1β to TNF-α is an indicator for predicting the prognosis of septic patients.

In one embodiment, when a blood concentration ratio of IL-1β to TNF-α is higher than a predetermined value, the blood concentration ratio may be an indicator of poor prognosis of a septic patient. The predetermined value means a value predetermined based on scientific evidence, in which the value may be any value as long as it is possible to determine whether or not a prognosis of a septic patient is poor based on the value as a reference. The predetermined value may vary depending on when the blood sample is taken from a patient, e.g., the elapsed time from the onset or diagnosis of sepsis, or hospitalization.

The predetermined value in the present invention can be set, for example, in a clinical trial or the like, based on the results of examining the relationship between the blood concentration ratio of IL-1β to TNF-α and prognosis for a plurality of patients suffering from sepsis at a predetermined time point (e.g., within 2 days, preferably within 1 day, from the onset or diagnosis of sepsis, or hospitalization). When there is a difference in prognosis between a group of patients in which the value of the blood concentration ratio is equal to or higher than a reference value and a group of patients in which the value of the blood concentration ratio is lower than the reference value, the reference value can be a predetermined value in the present invention.

In one embodiment, a higher blood concentration ratio of IL-1β to TNF-α in a particular septic patient compared to an average value of the blood concentration ratio of IL-1β to TNF-α in a group of septic patients with poor prognosis may indicate a poor prognosis for the particular septic patient.

The blood concentration ratio of IL-1β to TNF-α or the predetermined value in the present invention means a numerical value obtained by quantifying the measurement results of the blood concentrations of IL-1β and TNF-α in some way. The numerical value may be a value calculated using the value obtained from the measurement results (e.g., color intensity, fluorescence intensity, or the like) as it is, or a value calculated using a value obtained by separately preparing and quantifying a positive control sample having a known concentration. For example, when the blood concentrations of IL-1β and TNF-α are measured by the PEA method, the blood concentration ratio of IL-1β to TNF-α or predetermined values may be calculated from protein expression values (e.g., Normalized Protein eXpression (NPX)) obtained using a next-generation sequencer such as NovaSeq (Illumina, Inc.) or quantitative real-time PCR (qPCR) and analytical software such as NPX Explore software (Olink proteomics). In one embodiment, the blood concentration ratio of IL-1β to TNF-α may be calculated from protein expression values (e.g., NPX) measured by the PEA method. In a preferred embodiment, the blood concentration ratio of IL-1β to TNF-α may be calculated from protein expression values (e.g., NPX) measured by the PEA method and calculated using a next-generation sequencer and an analysis software (e.g., NPX Explore software (Olink proteomics)).

In certain embodiments, the predetermined value may be 1 or more, e.g., 2 or more, 3 or more, 4 or more, 5 or more, 6 or more, 7 or more, 8 or more, 9 or more, 10 or more, or more than these. Without wishing to be bound by any theory, when the blood concentration of IL-1β is higher than that of TNF-α, it is likely that the inflammasome pathway is activated and cytokine storm will occur to lead to septic shock. Thus, a blood concentration ratio of IL-1β to TNF-α of 1 or more, e.g., 2 or more, 3 or more, 4 or more, 5 or more, 6 or more, 7 or more, 8 or more, 9 or more, 10 or more, or more than these, may be an indicator of poor prognosis of the septic patient. In a preferred embodiment, the blood concentration ratio of IL-1β to TNF-α of 1 or more, e.g., 2 or more, 3 or more, 4 or more, 5 or more, 6 or more, 7 or more, 8 or more, 9 or more, 10 or more, or more than these, within 2 days, preferably within 1 day, from hospitalization, from an onset of sepsis, or from being diagnosed with sepsis may be an indicator of poor prognosis of the septic patient. In a more preferred embodiment, the blood concentration ratio of IL-1β to TNF-α of 1 or more, e.g., 2 or more, 3 or more, 4 or more, 5 or more, 6 or more, 7 or more, 8 or more, 9 or more, 10 or more, or more than these, within 2 days, preferably within 1 day, from hospitalization may be an indicator of poor prognosis of the septic patient.

The septic patient to be a subject in the present invention is a mammal, preferably a primate, more preferably a human.

In the present invention, a patient from which a blood sample is taken may not have been diagnosed with sepsis at the time the blood sample is taken, and may be, for example, a person who is suspected to suffer from sepsis. In addition, a patient from which a blood sample is taken may be a patient who has not yet received standard treatment for sepsis (see J-SSCG2020) or may be a patient who has already received a treatment, but preferably is a patient who has not received a treatment with an IL-6 inhibitor.

In another aspect, the present invention relates to the use of the biomarker I of the present invention described above for predicting a prognosis of a septic patient.

In one aspect, the present invention relates to a method for predicting a prognosis of a septic patient, the method comprising a step of measuring concentrations of IL-1β and TNF-α in a blood sample obtained from a septic patient, wherein a blood concentration ratio of IL-1β to TNF-α is an indicator for predicting the prognosis of the septic patient (hereinafter also referred to as the method I of the present invention).

In the method I of the present invention, the step of measuring concentrations of IL-1β and TNF-α can be performed according to the description about the biomarker I of the present invention.

In one embodiment, the method I of the present invention may further comprise calculating a blood concentration ratio of IL-1β to TNF-α from the measurement result obtained in the above step, and predicting the prognosis of the septic patient. As described above, a reference for predicting a prognosis of a septic patient can be appropriately set by those skilled in the art, and for example, when the blood concentration ratio of IL-1β to TNF-α is higher than a predetermined value, the prognosis of the septic patient can be predicted to be poor.

According to the present invention, the prognosis of a septic patient can be predicted, and, based on the results, a treatment plan (e.g., the need for administration of an IL-6 inhibitor, or the like) can be determined. For example, when the present invention predicts that a prognosis of a septic patient is poor, it is possible to improve the prognosis by an early treatment of the septic patient with an IL-6 inhibitor.

### 3. Biomarker for predicting therapeutic effect of IL-6 inhibitor in septic patient and use thereof

In one aspect, the present invention relates to a biomarker for predicting a therapeutic effect of an IL-6 inhibitor in a septic patient. The biomarker according to this aspect (hereinafter also referred to as the biomarker II of the present invention) comprises IL-1β and TNF-α, wherein a blood concentration ratio of IL-1β to TNF-α is an indicator for predicting a therapeutic effect of an IL-6 inhibitor in the septic patient.

As used herein, the "therapeutic effect" of an IL-6 inhibitor refers to a beneficial effect that results when an IL-6 inhibitor is administered to a septic patient. The therapeutic effect includes reduction or resolution of sepsis symptoms. The therapeutic effect also includes preventing the progression of sepsis symptoms, preventing or delaying the onset of septic shock, and reducing or resolving the symptoms of septic shock.

The septic patient to be a subject in the present invention and the acquisition of the blood concentration ratio of IL-1β to TNF-α are the same as in the biomarker I of the present invention and the method I of the present invention described in 2 above.

When the blood concentration ratio of IL-1β to TNF-α is higher than a predetermined value, the blood concentration ratio may be an indicator of expecting to obtain a therapeutic effect of an IL-6 inhibitor. The predetermined value means a value predetermined based on scientific evidence, in which the value may be any value as long as it is possible to determine whether or not a therapeutic effect of an IL-6 inhibitor is obtained based on the value as a reference. The predetermined value may vary depending on when the blood sample is taken from a patient, e.g., the elapsed time from the onset or diagnosis of sepsis, or hospitalization.

The predetermined value in the present invention can be set, for example, in a clinical trial or the like, based on the results of examining the relationship between the blood concentration ratio of IL-1β to TNF-α and the subsequent therapeutic effect of an IL-6 inhibitor for a plurality of patients suffering from sepsis at a predetermined time point (e.g., within 2 days, preferably within 1 day, from the onset or diagnosis of sepsis, or hospitalization). When there is a difference in the therapeutic effect of the IL-6 inhibitor between a group of patients in which the value of the blood concentration ratio is equal to or higher than a reference value and a group of patients in which the value of the blood concentration ratio is lower than the reference value, the reference value can be a predetermined value in the present invention.

In one embodiment, a higher blood concentration ratio of IL-1β to TNF-α in a particular septic patient compared to an average value of the blood concentration ratio of IL-1β to TNF-α in a group of septic patients for which a therapeutic effect of an IL-6 inhibitor has been obtained may indicate that the particular septic patient will obtain the therapeutic effect of the IL-6 inhibitor.

In another embodiment, a predetermined value in the present invention may be set in the same manner as in the biomarker I of the present invention. When the prognosis of a septic patient is predicted to be poor based on the blood concentration ratio of IL-1β to TNF-α, it is likely that cytokine storm will occur in the septic patient to lead to septic shock. For septic shock, a treatment with an IL-6 inhibitor is effective. Accordingly, the predetermined value in the biomarker I of the present invention may be applied as a predetermined value in the present invention.

The blood concentration ratio of IL-1β to TNF-α or a predetermined value in the present invention means a numerical value obtained by quantifying the measurement results of the blood concentrations of IL-1β and TNF-α in some way. The numerical value may be a value calculated using the value obtained from the measurement results (e.g., color intensity, fluorescence intensity, or the like) as it is, or a value calculated using a value obtained by separately preparing and quantifying a positive control sample having a known concentration. For example, when the blood concentrations of IL-1β and TNF-α are measured by the PEA method, the blood concentration ratio of IL-1β to TNF-α or predetermined values may be calculated from protein expression values (e.g., Normalized Protein eXpression (NPX)) obtained using a next-generation sequencer such as NovaSeq (Illumina, Inc.) or quantitative real-time PCR (qPCR) and analytical software such as NPX Explore software (Olink proteomics). In one embodiment, the blood concentration ratio of IL-1β to TNF-α may be calculated from protein expression values (e.g., NPX) measured by the PEA method. In a preferred embodiment, the blood concentration ratio of IL-1β to TNF-α may be calculated from protein expression values (e.g., NPX) measured by the PEA method and calculated using a next-generation sequencer and analysis software (e.g., NPX Explore software (Olink proteomics)).

In certain embodiments, the predetermined value may be 1 or more, e.g., 2 or more, 3 or more, 4 or more, 5 or more, 6 or more, 7 or more, 8 or more, 9 or more, 10 or more, or more than these. Without wishing to be bound by any theory, when the blood concentration of IL-1β is higher than that of TNF-α, it is likely that the inflammasome pathway is activated to lead to septic shock. Administration of an IL-6 inhibitor can prevent or delay the onset of septic shock and reduce or resolve symptoms of septic shock. Thus, a blood concentration ratio of IL-1β to TNF-α of 1 or more, e.g., 2 or more, 3 or more, 4 or more, 5 or more, 6 or more, 7 or more, 8 or more, 9 or more, 10 or more, or more than these, may be an indicator of expecting to obtain a therapeutic effect of an IL-6 inhibitor in the septic patient. In a preferred embodiment, the blood concentration ratio of IL-1β to TNF-α of 1 or more, e.g., 2 or more, 3 or more, 4 or more, 5 or more, 6 or more, 7 or more, 8 or more, 9 or more, 10 or more, or more than these, within 2 days, preferably within 1 day, from hospitalization, from an onset of sepsis, or from being diagnosed with sepsis may be an indicator of expecting to obtain a therapeutic effect of an IL-6 inhibitor in the septic patient. In a more preferred embodiment, the blood concentration ratio of IL-1β to TNF-α of 1 or more, e.g., 2 or more, 3 or more, 4 or more, 5 or more, 6 or more, 7 or more, 8 or more, 9 or more, 10 or more, or more than these, within 2 days, preferably within 1 day, from hospitalization may be an indicator of expecting to obtain a therapeutic effect of an IL-6 inhibitor in the septic patient.

In another aspect, the present invention relates to the use of the biomarker II of the present invention for predicting a therapeutic effect of an IL-6 inhibitor in a septic patient.

In one aspect, the present invention relates to a method for predicting a therapeutic effect of an IL-6 inhibitor in a septic patient, the method comprising a step of measuring concentrations of IL-1β and TNF-α in a blood sample obtained from a septic patient, wherein a blood concentration ratio of IL-1β to TNF-α is an indicator for predicting a therapeutic effect of an IL-6 inhibitor in the septic patient (hereinafter also referred to as the method II of the present invention).

In the method II of the present invention, the step of measuring concentrations of IL-1β and TNF-α can be performed according to the description about the biomarker II of the present invention.

In one embodiment, the method II of the present invention may further comprise calculating a blood concentration ratio of IL-1β to TNF-α from the measurement result obtained in the above step, and predicting a therapeutic effect of an IL-6 inhibitor in the septic patient. As described above, a reference for predicting a therapeutic effect of an IL-6 inhibitor in a septic patient can be appropriately set by those skilled in the art, and for example, when the blood concentration ratio of IL-1β to TNF-α is higher than a predetermined value, it can be predicted that the septic patient can obtain a therapeutic effect of an IL-6 inhibitor.

According to the present invention, a therapeutic effect of an IL-6 inhibitor in a septic patient can be predicted, and a treatment plan (e.g., a need for administration of an IL-6 inhibitor, or the like) can be determined based on the results. For example, when the present invention predicts that a septic patient can obtain a therapeutic effect of an IL-6 inhibitor, it is possible to obtain a therapeutic effect and improve the prognosis by an early treatment of the septic patient with an IL-6 inhibitor.

### 4. Biomarker for selecting septic patient who is expected to obtain therapeutic effect of IL-6 inhibitor and use thereof

In one aspect, the present invention relates to a biomarker for selecting a septic patient who is expected to obtain a therapeutic effect of an IL-6 inhibitor. The biomarker according to this aspect (hereinafter also referred to as the biomarker III of the present invention) comprises IL-1β and TNF-α, wherein a blood concentration ratio of IL-1β to TNF-α is an indicator of expecting to obtain a therapeutic effect of an IL-6 inhibitor in the septic patient.

The septic patient to be a subject in the present invention and the acquisition of the blood concentration ratio of IL-1β to TNF-α are the same as in the biomarker I of the present invention and the method I of the present invention described in 2 above.

When the blood concentration ratio of IL-1β to TNF-α is higher than a predetermined value, the blood concentration ratio may be an indicator of expecting to obtain a therapeutic effect of an IL-6 inhibitor. The predetermined value means a value predetermined based on scientific evidence, in which the value may be any value as long as it is possible to determine whether or not a therapeutic effect of an IL-6 inhibitor is obtained based on the value as a reference. The predetermined value may vary depending on when the blood sample is taken from a patient, e.g., the elapsed time from the onset or diagnosis of sepsis, or hospitalization.

The predetermined value in the present invention can be set, for example, in a clinical trial or the like, based on the results of examining the relationship between the blood concentration ratio of IL-1β to TNF-α and the subsequent therapeutic effect of an IL-6 inhibitor for a plurality of patients suffering from sepsis at a predetermined time point (e.g., within 2 days, preferably within 1 day, from the onset or diagnosis of sepsis, or hospitalization). When there is a difference in the therapeutic effect of the IL-6 inhibitor between a group of patients in which the value of the blood concentration ratio is equal to or higher than a reference value and a group of patients in which the value of the blood concentration ratio is lower than the reference value, the reference value can be a predetermined value in the present invention.

In one embodiment, a higher blood concentration ratio of IL-1β to TNF-α in a particular septic patient compared to an average value of the blood concentration ratio of IL-1β to TNF-α in a group of septic patients for which the therapeutic effect of the IL-6 inhibitor has been obtained may indicate that the particular septic patient will obtain the therapeutic effect of the IL-6 inhibitor.

In another embodiment, a predetermined value in the present invention may be set in the same manner as in the biomarker I of the present invention. When the prognosis of a septic patient is predicted to be poor based on the blood concentration ratio of IL-1β to TNF-α, it is likely that cytokine storm will occur in the septic patient to lead to septic shock. For septic shock, a treatment with an IL-6 inhibitor is effective. Accordingly, the predetermined value in the biomarker I of the present invention may be applied as a predetermined value in the present invention.

The blood concentration ratio of IL-1β to TNF-α or a predetermined value in the present invention means a numerical value obtained by quantifying the measurement results of the blood concentrations of IL-1β and TNF-α in some way. The numerical value may be a value calculated using the value obtained from the measurement results (e.g., color intensity, fluorescence intensity, or the like) as it is, or a value calculated using a value obtained by separately preparing and quantifying a positive control sample having a known concentration. For example, when the blood concentrations of IL-1β and TNF-α are measured by the PEA method, the blood concentration ratio of IL-1β to TNF-α or predetermined values may be calculated from protein expression values (e.g., Normalized Protein eXpression (NPX)) obtained using a next-generation sequencers such as NovaSeq (Illumina, Inc.) or quantitative real-time PCR (qPCR) and analytical software such as NPX Explore software (Olink proteomics). In one embodiment, the blood concentration ratio of IL-1β to TNF-α may be calculated from protein expression values (e.g., NPX) measured by the PEA method. In a preferred embodiment, the blood concentration ratio of IL-1β to TNF-α may be calculated from protein expression values (e.g., NPX) measured by the PEA method and calculated using a next-generation sequencer and analysis software (e.g., NPX Explore software (Olink proteomics)).

In certain embodiments, the predetermined value may be 1 or more, e.g., 2 or more, 3 or more, 4 or more, 5 or more, 6 or more, 7 or more, 8 or more, 9 or more, 10 or more, or more than these. Without wishing to be bound by any theory, when the blood concentration of IL-1β is higher than that of TNF-α, it is likely that the inflammasome pathway is activated to lead to septic shock. Administration of an IL-6 inhibitor can prevent or delay the onset of septic shock and reduce or resolve symptoms of septic shock. Thus, a blood concentration ratio of IL-1β to TNF-α of 1 or more, e.g., 2 or more, 3 or more, 4 or more, 5 or more, 6 or more, 7 or more, 8 or more, 9 or more, 10 or more, or more than these, may be an indicator of expecting to obtain a therapeutic effect of an IL-6 inhibitor in the septic patient. In a preferred embodiment, the blood concentration ratio of IL-1β to TNF-α of 1 or more, e.g., 2 or more, 3 or more, 4 or more, 5 or more, 6 or more, 7 or more, 8 or more, 9 or more, 10 or more, or more than these, within 2 days, preferably within 1 day, from hospitalization, from an onset of sepsis, or from being diagnosed with sepsis may be an indicator of expecting to obtain a therapeutic effect of an IL-6 inhibitor in the septic patient. In a more preferred embodiment, the blood concentration ratio of IL-1β to TNF-α of 1 or more, e.g., 2 or more, 3 or more, 4 or more, 5 or more, 6 or more, 7 or more, 8 or more, 9 or more, 10 or more, or more than these, within 2 days, preferably within 1 day, from hospitalization may be an indicator of expecting to obtain a therapeutic effect of an IL-6 inhibitor in the septic patient.

In another aspect, the present invention relates to the use of the biomarker III of the present invention for selecting a septic patient who is expected to obtain a therapeutic effect of an IL-6 inhibitor.

In one aspect, the present invention relates to a method for selecting a septic patient who is expected to obtain a therapeutic effect of an IL-6 inhibitor, the method comprising a step of measuring concentrations of IL-1β and TNF-α in a blood sample obtained from a septic patient, wherein a blood concentration ratio of IL-1β to TNF-α is an indicator of expecting to obtain a therapeutic effect of an IL-6 inhibitor in the septic patient (hereinafter also referred to as the method III of the present invention).

In the method III of the present invention, the step of measuring concentrations of IL-1β and TNF-α can be performed according to the description about the biomarker III of the present invention.

In one embodiment, the method III of the present invention may further comprise calculating a blood concentration ratio of IL-1β to TNF-α from the measurement result obtained in the above step, and selecting a septic patient who is expected to obtain a therapeutic effect of an IL-6 inhibitor based on the blood concentration ratio. As described above, a reference for selecting a septic patient can be appropriately set by those skilled in the art, and for example, when the blood concentration ratio of IL-1β to TNF-α is higher than a predetermined value, it can be predicted that the septic patient can obtain a therapeutic effect of an IL-6 inhibitor, and the septic patient can be selected as a subject for treatment with an IL-6 inhibitor.

The method III of the present invention may be a method that does not include medical practice. In this case, the method III of the present invention may be used to assist a physician, nurse, or other health care professionals in selecting a septic patient who is expected to obtain a therapeutic effect of an IL-6 inhibitor.

According to the present invention, a septic patient who is expected to obtain a therapeutic effect of an IL-6 inhibitor can be selected, and a treatment plan can be determined at an early stage. As a result, it is possible to improve the prognosis of the septic patient.

### 5. Therapeutic agent and method for sepsis

In one aspect, the present invention relates to a therapeutic agent for sepsis. As described above, since a biomarker for predicting a therapeutic effect of an IL-6 inhibitor in a septic patient and a biomarker for selecting a septic patient who is expected to obtain a therapeutic effect of an IL-6 inhibitor and methods of using the biomarkers are provided, the present invention also provides a therapeutic agent for sepsis, comprising an IL-6 inhibitor as an active ingredient, wherein the therapeutic agent is used to be administered to a septic patient selected based on a blood concentration ratio of IL-1β to TNF-α, who is expected to obtain a therapeutic effect of an IL-6 inhibitor (hereinafter also referred to as the therapeutic agent of the present invention).

In one embodiment, the IL-6 inhibitor according to the present invention can be a substance that binds to IL-6, a substance that binds to IL-6 receptor, or a substance that binds to gp130, preferably an anti-IL-6 receptor antibody, more preferably a chimeric anti-IL-6R antibody, a humanized anti-IL-6R antibody, or a human anti-IL-6R antibody, and further preferably tocilizumab or satralizumab.

The septic patient to be a subject to whom the therapeutic agent of the present invention is administered is a mammal, preferably a primate, more preferably a human.

The measurement of the blood concentration ratio of IL-1β to TNF-α in a septic patient can be performed as described in 2 above. The selection of a septic patient who is expected to obtain a therapeutic effect of an IL-6 inhibitor can be performed as described in 4 above.

In certain embodiments, the blood concentration ratio of IL-1β to TNF-α in a septic patient to be a subject to whom the therapeutic agent of the present invention is administered may be 1 or more, e.g., 2 or more, 3 or more, 4 or more, 5 or more, 6 or more, 7 or more, 8 or more, 9 or more, 10 or more, or more than these. In a preferred embodiment, the blood concentration ratio of IL-1β to TNF-α in a septic patient to be a subject to whom the therapeutic agent of the present invention is administered may be 1 or more, e.g., 2 or more, 3 or more, 4 or more, 5 or more, 6 or more, 7 or more, 8 or more, 9 or more, 10 or more, or more than these, within 2 days, preferably within 1 day, from hospitalization, from an onset of sepsis, or from being diagnosed with sepsis. In a more preferred embodiment, the blood concentration ratio of IL-1β to TNF-α in a septic patient to be a subject to whom the therapeutic agent of the present invention is administered may be 1 or more, e.g., 2 or more, 3 or more, 4 or more, 5 or more, 6 or more, 7 or more, 8 or more, 9 or more, 10 or more, or more than these, within 2 days, preferably within 1 day, from hospitalization.

The therapeutic agent of the present invention can be administered in the form of a medicament and can be administered systemically or topically, orally or parenterally. For example, intravenous injection such as infusion, intramuscular injection, intraperitoneal injection, subcutaneous injection, suppository, intestinal injection, oral enteric solvent, and the like can be selected, and the method of administration can be appropriately selected according to the age and symptoms of a patient. The effective dosage is not particularly limited, but ranges from 0.01 mg to 100 mg per kg of body weight per dose. Alternatively, the effective dosage can be selected from 1 to 1000 mg, preferably 5 to 50 mg per patient. Specific examples of the preferred dosage and administration method include, for example, in the case of an anti-IL-6 receptor antibody, the antibody is administered in a dosage of 0.5 mg to 40 mg, preferably 1 mg to 20 mg in one or several divided doses per month (4 weeks) per kg of body weight in a dosage schedule such as twice/week, once/week, once/2 weeks, once/4 weeks or the like by a method of intraperitoneal injection, intravenous injection such as infusion, subcutaneous injection, intramuscular injection, or the like. The dosage schedule can also be adjusted, for example, by extending the dosage interval from twice/week or once/week to once/2 weeks, once/3 weeks, once/4 weeks or the like while observing the patient's condition and observing the trend of blood test values.

The therapeutic agent of the present invention can contain a pharmaceutically acceptable carrier such as a preservative or stabilizer. The pharmaceutically acceptable carrier means a material that can be administered with the above agent. Examples of the pharmaceutically acceptable material include sterile water, saline, a stabilizer, an excipient, a buffer, a preservative, a surfactant, a chelating agent (such as EDTA), and a binder.

In the present invention, examples of the surfactant can include a non-ionic surfactant, and typical examples of the non-ionic surfactant can include sorbitan fatty acid esters such as sorbitan monocaprylate, sorbitan monolaurate, and sorbitan monopalmitate; glycerin fatty acid esters such as glycerin monocaprylate, glycerin monomyristate, and glycerin monostearate; polyglycerin fatty acid esters such as decaglyceryl monostearate, decaglyceryl distearate, and decaglyceryl monolinolate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, and polyoxyethylene sorbitan tristearate; polyoxyethylene sorbit fatty acid esters such as polyoxyethylene sorbit tetrastearate and polyoxyethylene sorbit tetraoleate; polyoxyethylene glycerin fatty acid esters such as polyoxyethylene glyceryl monostearate; polyethylene glycol fatty acid esters such as polyethylene glycol distearate; polyoxyethylene alkyl ether such as polyoxyethylene lauryl ether; polyoxyethylene polyoxypropylene alkyl ether such as polyoxyethylene polyoxypropylene glycol, polyoxyethylene polyoxypropylene propyl ether, and polyoxyethylene polyoxypropylene cetyl ether; polyoxyethylene alkyl phenyl ether such as polyoxyethylene nonyl phenyl ether; polyoxyethylene cured castor oil such as polyoxyethylene castor oil and polyoxyethylene cured castor oil (polyoxyethylene hydrogen castor oil); polyoxyethylene beeswax derivatives such as polyoxyethylene sorbitan beeswax; polyoxyethylene lanolin derivatives such as polyoxyethylene lanolin; and those having HLB 6 to 18 such as polyoxyethylene fatty acid amides, e.g., polyoxyethylene stearic acid amides.

Another example of the surfactant can include anionic surfactants, and typical examples of the anionic surfactants can include alkyl sulfates in which the alkyl group has 10 to 18 carbon atoms, such as sodium cetyl sulfate, sodium lauryl sulfate, and sodium oleyl sulfate; polyoxyethylene alkyl ether sulfates in which the average number of moles of ethylene oxide added is 2 to 4 and the alkyl group has 10 to 18 carbon atoms, such as sodium polyoxyethylene lauryl sulfate; alkyl sulfosuccinate ester salts in which the alkyl group has 8 to 18 carbon atoms, such as sodium lauryl sulfosuccinate ester; natural surfactants such as lecithin and glycerophospholipids; sphingophospholipid such as sphingomyelin; and sucrose fatty acid esters of fatty acids having 12 to 18 carbon atoms.

One or two or more of these surfactants can be added singly or in combination to the therapeutic agent of the present invention. Preferred surfactants for use in the formulation of the present invention are polyoxyethylene sorbitan fatty acid esters such as polysorbates 20, 40, 60 or 80, and particularly preferably polysorbates 20 and 80. Polyoxyethylene polyoxypropylene glycol represented by poloxamer (such as Pluronic F-68(R)) is also preferred.

The amount of surfactant added depends on the type of surfactant used, but in the case of polysorbate 20 or polysorbate 80, the amount is generally 0.001 to 100 mg/mL, preferably 0.003 to 50 mg/mL, and further preferably 0.005 to 2 mg/mL.

Examples of the buffer in the present invention can include phosphoric acid, citric acid buffer, acetic acid, malic acid, tartaric acid, succinic acid, lactic acid, potassium phosphate, gluconic acid, caprylic acid, deoxycholic acid, salicylic acid, triethanolamine, fumaric acid, other organic acids, or carbonic acid buffer, tris buffer, histidine buffer, and imidazole buffer.

The solution formulation may also be prepared by dissolving the therapeutic agent in an aqueous buffer known in the art of solution formulations. The concentration of the buffer is generally 1 to 500 mM, preferably 5 to 100 mM, and further preferably 10 to 20 mM.

The therapeutic agent of the present invention may contain other low molecular weight polypeptides, proteins such as serum albumin, gelatin and immunoglobulins, amino acids, sugars such as polysaccharides and monosaccharides, carbohydrates, and sugar alcohols.

The amino acids in the present invention can include basic amino acids such as arginine, lysine, histidine, or ornithine, or inorganic salts of these amino acids (preferably in the forms of hydrochlorides and phosphates, i.e. phosphate amino acids). When free amino acids are used, the preferred pH value is adjusted by the addition of an appropriate physiologically acceptable buffering substance, such as an inorganic acid, in particular hydrochloric acid, phosphoric acid, sulfuric acid, acetic acid, formic acid, or a salt thereof. In this case, the use of phosphates is particularly advantageous in that an especially stable lyophilized product can be obtained. It is particularly advantageous when the preparation is substantially free of organic acids, such as malic acid, tartaric acid, citric acid, succinic acid, fumaric acid, and the like, or when there are no corresponding anions (malic acid ions, tartaric acid ions, citric acid ions, succinic acid ions, fumaric acid ions, and the like) therein. Preferred amino acids are arginine, lysine, histidine, or ornithine. In addition, acidic amino acids such as glutamic acid and aspartic acid, and salt forms (preferably sodium salts) thereof, or neutral amino acids, such as isoleucine, leucine, glycine, serine, threonine, valine, methionine, cysteine, or alanine, or aromatic amino acids, such as phenylalanine, tyrosine, tryptophan, or a derivative N-acetyl tryptophan, can also be used.

In the present invention, examples of the sugars such as polysaccharides and monosaccharides and the carbohydrates include dextran, glucose, fructose, lactose, xylose, mannose, maltose, sucrose, trehalose, and raffinose.

In the present invention, examples of the sugar alcohols can include mannitol, sorbitol, and inositol.

When the therapeutic agent of the present invention is prepared as an aqueous solution for injection, it can be mixed with an isotonic solution containing, for example, saline, glucose or other adjuvants (e.g., D-sorbitol, D-mannose, D-mannitol, sodium chloride), and the aqueous solution may be used in conjunction with an appropriate dissolution aid (e.g., alcohol such as ethanol, polyalcohol such as propylene glycol or PEG, and nonionic surfactant such as polysorbate 80 or HCO-50).

Optionally, the therapeutic agent of the present invention may further contain a diluent, a dissolution aid, a pH adjuster, a painless agent, a sulfur-containing reducing agent, an antioxidant, and the like.

Examples of the sulfur-containing reducing agent in the present invention can include those having a sulfhydryl group such as N-acetylcysteine, N-acetyl homocysteine, thioctic acid, thiodiglycol, thioethanolamine, thioglycerol, thiosorbitol, thioglycolic acid and a salt thereof, sodium thiosulfate, glutathione, and thioalkanoic acid having 1 to 7 carbon atoms.

Examples of the antioxidant in the present invention can include erythorbic acid, dibutylhydroxytoluene, butylhydroxyanisole, α-tocopherol, tocopherol acetate, L-ascorbic acid and a salt thereof, L-ascorbic acid palmitate, L-ascorbic acid stearate, sodium bisulfite, sodium sulfite, triamyl gallate, propyl gallate, or chelating agents such as ethylenediamine disodium tetraacetate (EDTA), sodium pyrophosphate, or sodium metaphosphate.

The therapeutic agent of the present invention can also be encapsulated in microcapsules such as microcapsules of hydroxymethylcellulose, gelatin, or poly[methyl methacrylic acid], or formulated in colloidal drug delivery systems such as liposomes, albumin microspheres, microemulsions, nanoparticles, or nanocapsules, as needed (see "Remington's Pharmaceutical Science 16th edition", Oslo Ed., 1980, and the like). In addition, methods for formulating an agent to a sustained-release agent are known and may be applied to the present invention (Langer et al., J. Biomed. Mater. Res. 1981, 15: 167-277; Langer, Chem. Tech. 1982, 12: 98-105;U.S. Patent No. 3,773,919; European Patent (EP) Application Publication No. 58,481; Sidman et al., Biopolymers 1983, 22: 547-556; EP Patent No. 133,988).

The pharmaceutically acceptable carrier used is suitably selected singly or in combination from the above materials depending on the dosage form, but are not limited to these.

In another aspect, the present invention relates to the use of the therapeutic agent of the present invention.

In one aspect, the present invention relates to a method for treating sepsis, the method comprising administering an IL-6 inhibitor to a septic patient, wherein the septic patient is a septic patient selected based on a blood concentration ratio of IL-1β to TNF-α, who is expected to obtain a therapeutic effect of an IL-6 inhibitor (hereinafter also referred to as the treating method of the present invention).

In the treating method of the present invention, the IL-6 inhibitor, the septic patient to be a subject, the blood concentration ratio of IL-1β and its measurement, and the selection of the septic patient are the same as in the therapeutic agent of the present invention.

In one embodiment, the treating method of the present invention may further comprise a step of measuring concentrations of IL-1β and TNF-α in a blood sample obtained from a septic patient. In this embodiment, the treating method of the present invention may further comprise a step of calculating a blood concentration ratio of IL-1β to TNF-α from a measurement result obtained in the above step, and selecting a septic patient who is expected to obtain a therapeutic effect of an IL-6 inhibitor based on the blood concentration ratio. These steps can be performed according to the description of the method III of the present invention. As described above, a reference for selecting a septic patient can be appropriately set by those skilled in the art, and for example, when the blood concentration ratio of IL-1 β to TNF-α is higher than a predetermined value, it can be predicted that the septic patient can obtain a therapeutic effect of an IL-6 inhibitor, and the septic patient can be selected as a subject for treatment with an IL-6 inhibitor.

According to the present invention, it is possible to provide an appropriate treatment at an early stage to a septic patient who is expected to obtain a therapeutic effect of an IL-6 inhibitor, and to improve the prognosis of the septic patient.

### 6. suppressor and suppression method of cytokine storm associated with sepsis

In one aspect, the present invention relates to a suppressor of cytokine storm associated with sepsis. As described above, since when the blood concentration of IL-1β is higher than that of TNF-α, it is likely that the inflammasome pathway is activated and cytokine storm will occur to lead to septic shock, the present invention relates to a suppressor of cytokine storm associated with sepsis, the suppressor comprising an IL-6 inhibitor as an active ingredient, wherein the suppressor is used to be administered to a septic patient selected based on a blood concentration ratio of IL-1β to TNF-α, who is expected to obtain a therapeutic effect of an IL-6 inhibitor (hereinafter also referred to as the suppressor of the present invention).

In the suppressor of the present invention, the IL-6 inhibitor, the septic patient to be a subject, the blood concentration ratio of IL-1β and its measurement, and the selection of the septic patient are the same as in the therapeutic agent of the present invention. In addition, the administration form, dose, dosage, and the like of the suppressor of the present invention are the same as in the therapeutic agent of the present invention.

In another aspect, the present invention relates to the use of the suppressor of the present invention.

In one aspect, the present invention relates to a method for suppressing cytokine storm associated with sepsis, the method comprising administering an IL-6 inhibitor to a septic patient, wherein the septic patient is a septic patient selected based on a blood concentration ratio of IL-1β to TNF-α, who is expected to obtain a therapeutic effect of the IL-6 inhibitor (hereinafter also referred to as the suppression method of the present invention).

In the suppression method of the present invention, the IL-6 inhibitor, the septic patient to be a subject, the blood concentration ratio of IL-1β and its measurement, and the selection of the septic patient are the same as in the treating method of the present invention.

In one embodiment, the suppression method of the present invention may further comprise a step of measuring concentrations of IL-1β and TNF-α in a blood sample obtained from a septic patient. In this embodiment, the treating method of the present invention may further comprise calculating a blood concentration ratio of IL-1β to TNF-α from the measurement result obtained in the above step, and selecting a septic patient who is expected to obtain a therapeutic effect of an IL-6 inhibitor based on the blood concentration ratio. These steps can be performed according to the description of the method III of the present invention. As described above, a reference for selecting a septic patient can be appropriately set by those skilled in the art, and for example, when the blood concentration ratio of IL-1β to TNF-α is higher than a predetermined value, it can be predicted that the septic patient can obtain a therapeutic effect of an IL-6 inhibitor, and the septic patient can be selected as a subject for treatment with an IL-6 inhibitor.

According to the present invention, it is possible to suppress cytokine storms by providing an appropriate treatment at an early stage to a septic patient who is expected to obtain a therapeutic effect of an IL-6 inhibitor, and to improve the prognosis of the septic patient.

### 7. Kit

In one aspect, the present invention relates to a kit for predicting a prognosis of a septic patient, for predicting a therapeutic effect of an IL-6 inhibitor in a septic patient, or for selecting a septic patient who is expected to obtain a therapeutic effect of an IL-6 inhibitor, the kit comprising a reagent for measuring blood concentrations of IL-1β and TNF-α and calculating a blood concentration ratio of IL-1β to TNF-α.

The reagent comprised in the kit of the present invention are not particularly limited as long as blood concentrations of IL-1β and TNF-α can be measured, and the reagents can be appropriately selected according to the measuring method of the blood concentrations. Examples of the reagent include antibodies or antibody fragments that specifically bind to each of IL-1β and TNF-α. The kit of the present invention may comprise known reagents depending on the measuring method of the blood concentrations, for example, reagents for enzyme immunoassays (ELISA, EIA), fluorescence immunoassays (FIA), radioimmunoassays (RIA), luminescence immunoassays (LIA), electrochemiluminescence (ECL) methods, western blotting methods, surface plasmon resonance methods, methods using antibody arrays, immunohistostaining methods, fluorescence activated cell sorting (FACS) methods, immunochromatography methods, immunoprecipitation methods, immunonephelometry methods, latex agglutination methods, and Proximity Extension Assay (PEA) methods, such as labeling reagents, buffers, chromogenic substrates, secondary antibodies, blocking agents, instruments necessary for testing, and controls.

The kit of the present invention may also comprise a positive control sample that serves as a reference when measuring blood concentrations of IL-1β and TNF-α. The positive control sample is not particularly limited as long as the amounts of IL-1β and TNF-α contained therein are specified in advance, and can be prepared as appropriate according to the measuring method of blood concentration. The positive control sample may be, for example, a sample containing isolated, purified and quantified IL-1β and TNF-α.

The kit of the present invention may further comprise an instruction describing a use method of the kit. Such a use method may include a method for predicting a prognosis of a septic patient, a method for predicting a therapeutic effect of an IL-6 inhibitor in a septic patient, or a method for selecting a septic patient who is expected to obtain a therapeutic effect of an IL-6 inhibitor, from a blood concentration ratio of IL-1β to TNF-α calculated from blood concentrations of IL-1β and TNF-α. For example, the instruction may state that, when the blood concentration ratio of IL-1β to TNF-α in a sample obtained from a septic patient is higher than a preset predetermined value, it is determined that the prognosis of the septic patient is poor or the septic patient is expected to obtain a therapeutic effect of an IL-6 inhibitor.

Note that all cited literatures cited herein are incorporated herein by reference.

The present invention will be described in more detail by way of Examples, but the present invention is not limited thereto. Various changes and modifications are possible for those skilled in the art, and these changes and modifications are also included in the present invention.

### EXAMPLES

### I. Method

### 1. Target Population

Among the patients hospitalized in the Department of Traumatology and Acute Critical Medicine at Osaka University (https://www.osaka-u-tacec.com/), those diagnosed with sepsis by Sepsis-3¹⁾ were targeted. It should be noted that treatment with IL-6 inhibitors for septic patients has not been approved, and thus no IL-6 inhibitors were administered to the target population.

### 2. Evaluation of Death28

(1) At Osaka University, the presence or absence of day-28 death (Death28)²⁾, which is commonly used as a clinical outcome for sepsis, was evaluated as a prognostic indicator for patients of the target population.
(2) The data about the presence or absence of Death28 was sent to CHUGAI PHARMACEUTICAL CO., LTD.

### 3. Samples

The samples used were serum samples (176 samples) separated from peripheral blood collected from patients (38 cases) among the target population, from whom consents were appropriately obtained.

Serum samples collected from 2014 to 2015 were cryopreserved at Osaka University, and subjected to the treatment of 4(1) described below at GeneticLab Co., Ltd. (https://www.gene-lab.com/) in 2021, and then subjected to the serum biomarker measurement of 4(2) described below at OlinkProteomics Inc. (https://www.bioxpedia.com/olink-proteomics/).

### 4. Serum biomarker (BM) measurement

(1) Frozen serum samples were sent from Osaka University to GeneticLab Co., Ltd. while being cooled with dry ice. After freeze-thawing, the samples were subjected to the following sample processing and preparation.
   i) Preparation of 10% Triton X-100 stock: 1 mL of Triton X-100 was added to 9 mL of PBS and mixed well. After preparation, the mixture was stored at 4°C and the expiration date was set at 7 days.
   ii) 5 µL of 10% Triton X-100 stock was placed in a 1.5 mL tube.
   iii) Serum was added by 45 µL each.
   iv) They were mixed using vortex and spun down at 400 x g for 1 min.
   v) The mixture was stood still as it was at room temperature (18°C to 25°C) for 2 hours.
   vi) The resultant was stored in a sample storage deep freezer until shipped.
(2) The frozen samples were sent from GeneticLab Co., Ltd. to the Uppsala lab of OlinkProteomics Inc. while being cooled with dry ice. Then, the samples were comprehensively measured for about 1,500 biomarkers including serum IL-1β and TNF-α, which were related to inflammation, cardiovascular metabolism, nervous system, and cancer, by the following procedure.
(2-1) Preparation of plate for measurement
   A) 40 µL or more of each sample was used as a sample to be added to the wells of B).
   B) A PCR-clean 96-well PCR plate, preferably one with a full skirt (e.g., Thermo Fisher Scientific #AB0800 with MicroAmp seal #4306311 from Thermo Fisher Scientific Inc.) was used.
   C) Each well was confirmed to be sealed separately using an adhesive film or a separate seal.
(2-2) Measurement using Olink(R) Explore
   A) A measurement was performed with Explore 1536 (including all four panels of Explore 384 inflammation, Explore 384 cardiometabolic, Explore 384 neurology, and Explore 384 oncology). Specifically, an immunoassay was performed with two DNA-tagged antibodies for one protein using the Proximity Extension Assay (PEA) method, and the expression was quantified based on the resulting double-stranded DNA, which was formed by binding of the two antibodies to the protein, with a next-generation sequencer (NGS) (Olink, White Paper "PEA - a high-multiplex immunoassay technology with qPCR or NGS readout" (https://olink.com/resources-support/document-download-center/#whitepapers)).
(2-3) The measured values of IL-1β and TNF-α (semiquantitative values in units of Normalized Protein eXpression (NPX)) were sent from OlinkProteomics Inc. to CHUGAI PHARMACEUTICAL CO., LTD.

### 5. Analysis

(1) At CHUGAI PHARMACEUTICAL CO., LTD., death28 and serum IL-1β and TNF-α values sent in 2(2) and 4(2-3) above were analyzed with SAS program (SAS9.4 for Windows (SAS Institute Inc., NC, USA)). For Death28, stratified analysis was performed on patients who died (Y) and survived (N) within 28 days after hospitalization. For IL-1β and TNF-α, the values obtained from OlinkProteomics Inc. (semiquantitative values of Log2 scale in units of Normalized Protein eXpression (NPX)) were converted by 2^{NPX} = linear NPX for linear scale, and a value of IL-1β/TNF-α was calculated.

### II. Results

The analysis results are shown in Fig. 1 and Table 1. The ratio of IL-1β/TNF-α on day 1 of hospitalization was 40.1 (± 80.4) (mean (± SD), the same hereinafter) and 1.86 (± 2.51) for patients who died (N = 6) and those who survived (N = 28) within 28 days after hospitalization, respectively, showing a tendency that the ratio for patients who died within 28 days after hospitalization was higher. Accordingly, it was shown that the blood concentration ratio of IL-1 β to TNF-α is useful as a marker for predicting a prognosis of sepsis.

**[Table 1]**

| | | | | | | | | | |
|---|---|---|---|---|---|---|---|---|---|
| Disease: Sepsis | | | | | | | | | |
| Parameter: IL-1β/TNF-α ratio | | | | | | | | | |
| Sampling date: Day 1 | | | | | | | | | |

| DEATH28 | N | Mean | SD | CV (%) | Min | 25%ile | Median | 75%ile | Max |
|---|---|---|---|---|---|---|---|---|---|
| NO | 28 | 1.86 | 2. 51 | 135 | 0.0177 | 0. 227 | 0. 673 | 2. 47 | 8.65 |
| YES | 6 | 40.1 | 80.4 | 201 | 2.45 | 3.1 | 8.32 | 14.4 | 204 |
| SD: Standard deviation; CV: Coefficient of variation; | | | | | | | | | |
| 25%ile: 25 percentile; 75%ile: 75 percentile | | | | | | | | | |

### III. References

1) Singer M, Deutschman CS, Seymour CW, et al: The third international consensus definitions for sepsis and septic shock (Sepsis-3). JAMA. 2016; 315: 801-10.doi: 10.1001/jama.2016.0287.
2) The Japanese Clinical Practice Guidelines for Management of Sepsis and Septic Shock 2020 (J-SSCG 2020); The Journal of the Japanese Society of Intensive Care Medicine Vol. 28 Supplement, February 25, 2021

### INDUSTRIAL APPLICABILITY

According to the present invention, more effective treatments are provided for septic patients, especially those whose prognosis is predicted to be poor.

## Claims

1. A method for predicting a prognosis of a septic patient, the method comprising
a step of measuring concentrations of IL-1β and TNF-α in a blood sample obtained from a septic patient, wherein
a blood concentration ratio of IL-1β to TNF-α is an indicator for predicting a prognosis of the septic patient.

2. The method according to claim 1, wherein the blood concentration ratio of 1 or more is an indicator of poor prognosis of the septic patient.

3. The method according to claim 1 or 2, wherein the blood concentration ratio is a blood concentration ratio of IL-1β to TNF-α within 2 days, preferably within 1 day, from hospitalization, from an onset of sepsis, or from being diagnosed with sepsis.

4. The method according to any one of claims 1 to 3, wherein the blood sample is serum, plasma, or whole blood.

5. The method according to any one of claims 1 to 4, wherein the blood concentrations of IL-1β and TNF-α are measured by a Proximity Extension Assay (PEA) method.

6. The method according to claim 5, wherein a measured value by the PEA method is an expression value of a protein obtained using a next-generation sequencer or quantitative real-time PCR.

7. The method according to any one of claims 1 to 6, wherein the septic patient is a human.

8. The method according to any one of claims 1 to 7, wherein the septic patient has not received a treatment with an IL-6 inhibitor.

9. The method according to any one of claims 1 to 8, wherein the prognosis is a life prognosis.

10. A biomarker for predicting a prognosis of a septic patient, the biomarker comprising IL-1β and TNF-α, wherein
a blood concentration ratio of IL-1β to TNF-α is an indicator for predicting a prognosis of the septic patient.

11. The biomarker according to claim 10, wherein the blood concentration ratio of 1 or more is an indicator of poor prognosis of the septic patient.

12. The biomarker according to claim 10 or 11, wherein the blood concentration ratio is a blood concentration ratio of IL-1β to TNF-α within 2 days, preferably within 1 day, from hospitalization, from an onset of sepsis, or from being diagnosed with sepsis.

13. The biomarker according to any one of claims 10 to 12, wherein blood concentrations of IL-1β and TNF-α are concentrations of IL-1β and TNF-α in serum, plasma, or whole blood obtained from the septic patient.

14. The biomarker according to any one of claims 10 to 13, wherein the blood concentrations of IL-1β and TNF-α are measured by a Proximity Extension Assay (PEA) method.

15. The biomarker according to claim 14, wherein a measured value by the PEA method is an expression value of a protein obtained using a next-generation sequencer or quantitative real-time PCR.

16. The biomarker according to any one of claims 10 to 15, wherein the septic patient is a human.

17. The biomarker according to any one of claims 10 to 16, wherein the septic patient has not received a treatment with an IL-6 inhibitor.

18. The biomarker according to any one of claims 10 to 17, wherein the prognosis is a life prognosis.
